# EUROPEAN PATENT APPLICATION

(11) **EP 4 105 240 A1**
(43) Date of publication of application: **21.12.2022**
(21) Application number: 21753498.1
(22) Date of filing: 05.02.2021
(51) Int. Cl.: C07K 19/00, C12N 5/10, C12N 15/12, A61K 35/17, A61K 48/00, A61P 35/00, A61P 37/02

(54) **CHIMERIC ANTIGEN RECEPTOR AND USE THEREOF**

(30) Priority: 13.02.2020 CN 202010090749
(71) Applicant: Sichuan University, Sichuan 610065 (CN)
(72) Inventor: WANG, Wei, Chengdu, Sichuan 610065 (CN); WEI, Yuquan, Chengdu, Sichuan 610065 (CN)
(74) Representative: Dolphin, Kirsty Mairi
(86) International application number: PCT/CN2021/075544
(87) International publication number: WO 2021/160038

(57) **Abstract**

The present invention belongs to the field of biomedicine, and particularly relates to a chimeric antigen receptor and use thereof. The present invention provides a novel chimeric antigen receptor, comprising an extracellular domain, a transmembrane domain and an intracellular domain, wherein the transmembrane domain and the intracellular domain of the chimeric antigen receptor form a costimulatory signaling domain, and the costimulatory signaling domain comprises a full length or a fragment of an amino acid sequence encoding a reverse dectin-1. Experiments prove that novel CAR-T cells provided by the present invention are effective against a variety of solid tumors and hematological malignancies.

## Description

### Priority Application

. The present application claims priority from Chinese invention patent application CN2020100907495 "A COSTIMULATORY SIGNALING DOMAIN OF A CHIMERIC ANTIGEN RECEPTOR AND USE THEREOF" filed on February 13, 2020, which is incorporated by reference in its entirety.

### Technical Field

. The present invention belongs to the field of biomedicine, and particularly relates to a chimeric antigen receptor and use thereof.

### Background

.Chimeric antigen receptor (CAR) T cell immunotherapy, as a promising tumor therapy strategy, has undergone a series of evolutionary processes. T cell activation mediated by a 1st generation CAR is accomplished by tyrosine-based activation motifs on CD3v or FceRIg. However, antitumor activity of T cells engineered by the 1st generation CAR is limited in vivo, resulting in T cell apoptosis due to reduced T cell proliferation. A 2nd generation CAR is added with a new intracellular costimulatory signal, such as 4-1BB or CD28. Compared with the 1st generation CAR, the 2nd generation CAR shows unchanged antigen specificity, increased T cell proliferation and cytokine secretion, increased anti-apoptotic protein secretion, and delayed cell death. Existing CAR-T cell immunotherapy has achieved remarkable progress in treating hematological malignancies, but limited success was observed in treating solid tumors.

. The progress of the CAR-T cell immunotherapy in treating hematological malignancies includes treating non-Hodgkin lymphoma (NHL), B-cell acute lymphoblastic leukemia (ALL), multiple myeloma (MM) and chronic lymphoblastic leukemia (CLL). So far, the objective response rate (ORR) in CD19-specific or BCMA-specific CAR-T clinical trials ranges from 48% to 95%. Two CAR-T cell products, specific to the B lymphoma, Axicabtagene Ciloleucel (KTE-C19, Kite Pharma) and Tisagenlecleuce (CTL019, Novartis), were approved by the U.S. Food and Drug Administration (US FDA) in 2017. In a phase 1/2 trial with Axicabtagene ciloleucel, 2-year follow-up data, involving 108 patients with the refractory large B-cell lymphoma, showed that 83% of patients had an objective response (OR), and 58% of them had a complete response (CR), with a median follow-up of 15.4 months (IQR 13.7-17.3). This suggests that CAR-T cell therapy can maintain a long-term remission. But, there are a lot of challenges in the application of CAR-T cell therapy in the solid tumors, such as lack of appropriate tumor-specific antigen, inhibition of tumor microenvironment, and insufficient CAR-T cell localization and persistence. In addition, continuous antigen exposure can result in CAR-T cell exhaustion, and then compromising the effectiveness of CAR-T cells against tumors. Therefore, new approaches are necessary to design CARs in the treatment of solid tumors.

. Dectin-1, i.e. CLEC-7A, is a new subgroup of C-type lectin receptors (CLRs). Dectin-1 comprises an extracellular domain, a transmembrane domain and an intracellular domain. The extracellular domain of dectin-1 has been utilized as the scFv of CAR-T cells to target fungus. Dectin-1 is not only predominantly expressed on myeloid cells, including neutrophils, monocytes, dendritic cells and macrophages, but also on some subsets of human T cells and B cells. Dectin-1 plays an important role in tumor growth and metastasis by activating the NK cells. Dectin-1 can also regulate various cellular responses, such as DC maturation, antigen presentation and production of cytokine and chemokine. In addition, dectin-1 can directly induce innate immune memory, and influence the development of CD8, CD4, T and B cells in theory.

. In conclusion, the experiment team of the present invention designed a novel 2nd generation CAR-T cell engineered by incorporation of dectin-1 into a 2nd generation CAR structure as a costimulatory signaling domain and assessed function and antitumor activity of the engineered novel 2nd generation CAR-T cell, and thus to address at least one of the above problems.

### Summary

. In view of this, one of objectives of the present invention is to provide a novel 2nd generation CAR structure.

. A chimeric antigen receptor, comprising an extracellular domain, a transmembrane domain and an intracellular domain, wherein the transmembrane domain and the intracellular domain of the chimeric antigen receptor form a costimulatory signaling domain, and the costimulatory signaling domain comprises a full length or a fragment of an amino acid sequence encoding a reverse dectin-1.

. Further, the extracellular domain comprises a single-chain antibody targeting to CD19 or HER2.

. Further, the chimeric antigen receptor further comprises a CD8α hinge region, a reverse dectin-1 transmembrane domain, a reverse dectin-1 intracellular signaling domain and a CD3ζ intracellular signaling domain which are connected in sequence.

. Further, the amino acid sequence encoding the reverse dectin-1 is shown in SEQ ID NO:3.

. Further, an amino acid sequence encoding the reverse dectin-1 transmembrane domain is shown in SEQ ID NO:2.

. Further, an amino acid sequence encoding the reverse dectin-1 intracellular signaling domain is shown in SEQ ID NO:1.

. A lentiviral vector comprising any one of the above chimeric antigen receptors.

. Further, the lentiviral vector comprises pCLK, psPAX2 or pMD2.0G.

. One of objectives of the present invention is also to provide a CAR-T cell and use thereof, wherein the CAR-T cell is engineered by the above novel 2nd generation CAR.

. A CAR-T cell expressing the above chimeric antigen receptor.

. An antitumor drug, comprising the above CAR-T cell and a pharmaceutically acceptable excipient and/or adjuvant.

. Further, the tumor comprises hematological tumors and solid tumors.

. Further, the tumor comprises large B-cell lymphoma, B-cell lymphoma, non-Hodgkin lymphoma, myelogenous leukemia, lymphoblastic leukemia, breast cancer, gastric cancer, esophageal cancer or ovarian cancer.

. Use of the above CAR-T cell in preparation of an antitumor drug.

. Further, the CAR-T cell can stimulate secretion of effector cytokine.

. Further, the effector cytokine comprises IFN-γ, TNF-α and IL-6.

. Further, the CAR-T cell, upon being stimulated, display phenotype of a central memory T cell.

. Further, the tumor comprises hematological tumors and solid tumors.

. Further, the tumor comprises large B-cell lymphoma, B-cell lymphoma, non-Hodgkin lymphoma, myelogenous leukemia, lymphoblastic leukemia, breast cancer, gastric cancer, esophageal cancer or ovarian cancer.

. The objective of the present invention is also to provide a synthesis method for the above chimeric antigen receptor.

. A synthesis method for the above chimeric antigen receptor, comprising the following steps: (1) synthesizing a gene sequence of the reverse dectin-1 transmembrane domain - reverse dectin-1 intracellular domain or a gene sequence of the reverse dectin-1 transmembrane domain - CD3ζ intracellular signaling domain; (2) synthesizing primers according to a target, and synthesizing a gene sequence of the chimeric antigen receptor by an overlapping PCR method.

. Further, the step (2) specifically comprises: first using primers F1 and R1 to expand a bound gene sequence of the extracellular domain and the CD8α hinge region, then using primers F2 and R2 to expand the gene sequence of the reverse dectin-1 transmembrane domain - CD3ζ intracellular signaling domain, and finally using the bound gene sequence of the extracellular domain and the CD8α hinge region as well as the gene sequence of the reverse dectin-1 transmembrane domain - CD3ζ intracellular signaling domain as templates and using F1 and R2 as primers to synthesize the gene sequence of the chimeric antigen receptor.

. Further, the primer F1 is shown in SEQ ID NO:7, R1 is shown in SEQ ID NO:8, F2 is shown in SEQ ID NO:9, and R2 is shown in SEQ ID NO:10.

. A method for synthesizing a gene sequence of the reverse dectin-1 transmembrane domain - reverse dectin-1 intracellular domain is the same.

### Beneficial Effects

. The present invention provides a novel 2nd generation CAR structure using a reverse dectin-1 as a costimulatory signaling domain and a CAR-T cell prepared thereby. In vivo and in vitro experiment results of the present invention reveal that the novel CAR design influences T cell functions through dectin-1 costimulation, such as enhanced cytokine secretion and lytic capacity of a variety of cytokines (including IFN-γ, TNF-α and IL-6), reduced exhaustion potential, increased cell expansion and distinct antitumor activity. Experiments prove that novel CAR-T cells provided by the present invention are effective against a variety of solid tumors and hematological malignancies.

### Description of Drawings

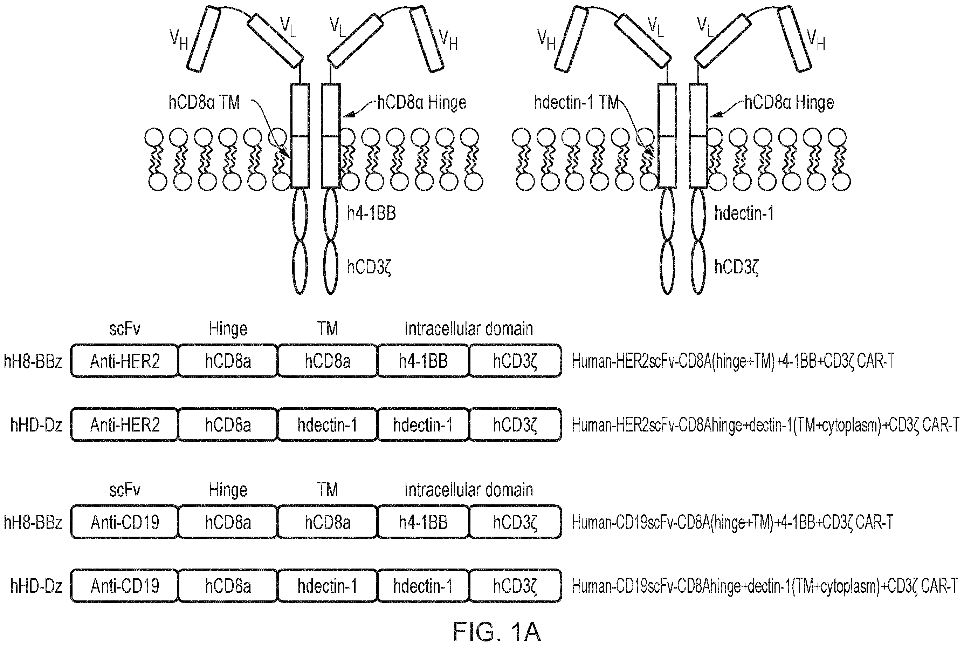

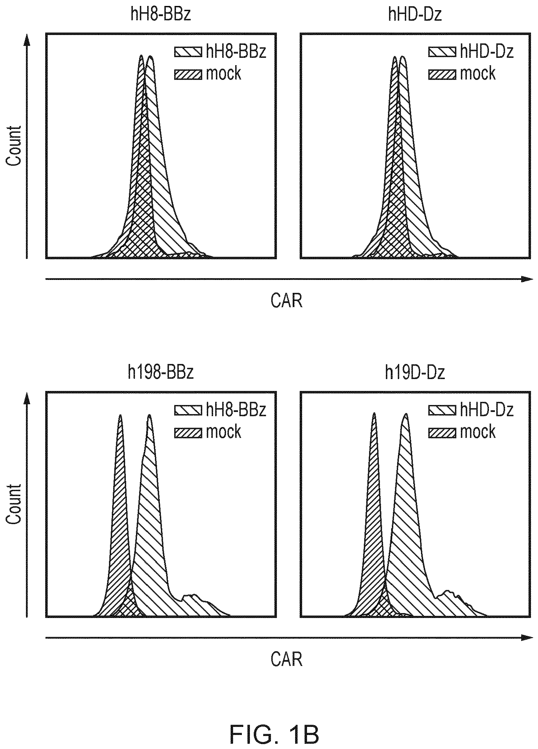

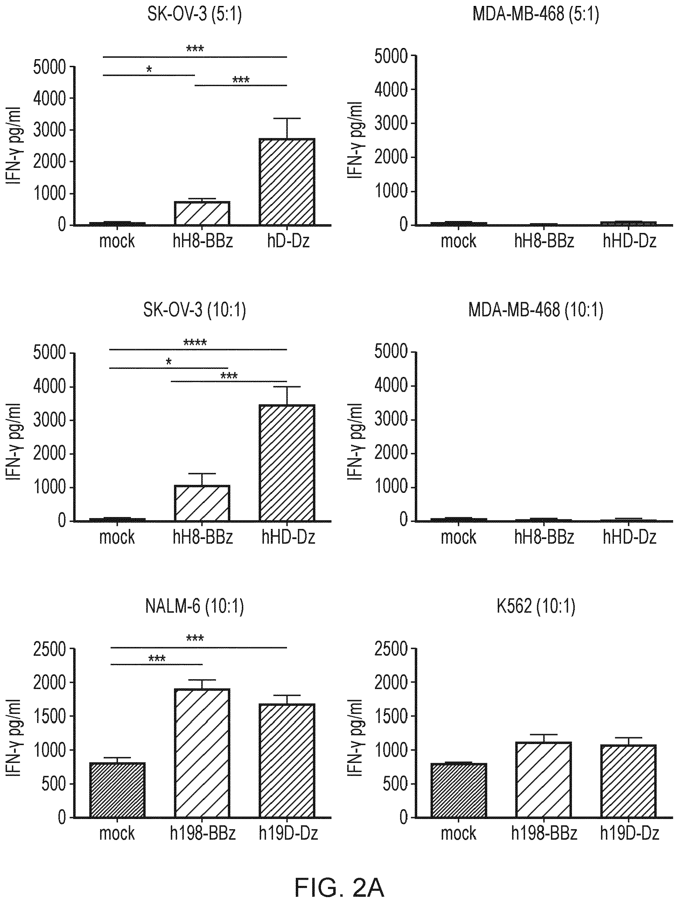

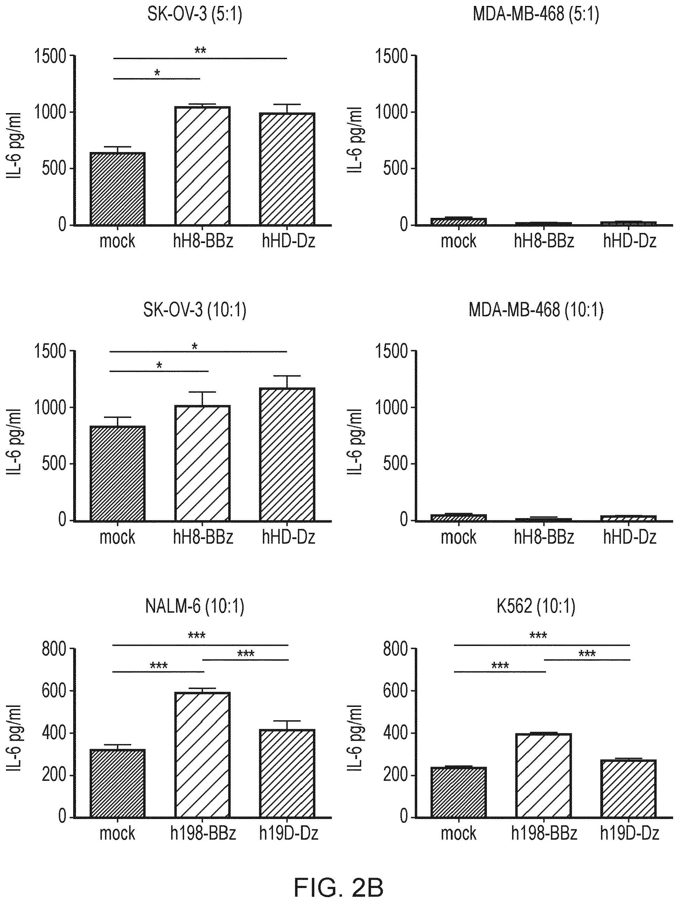

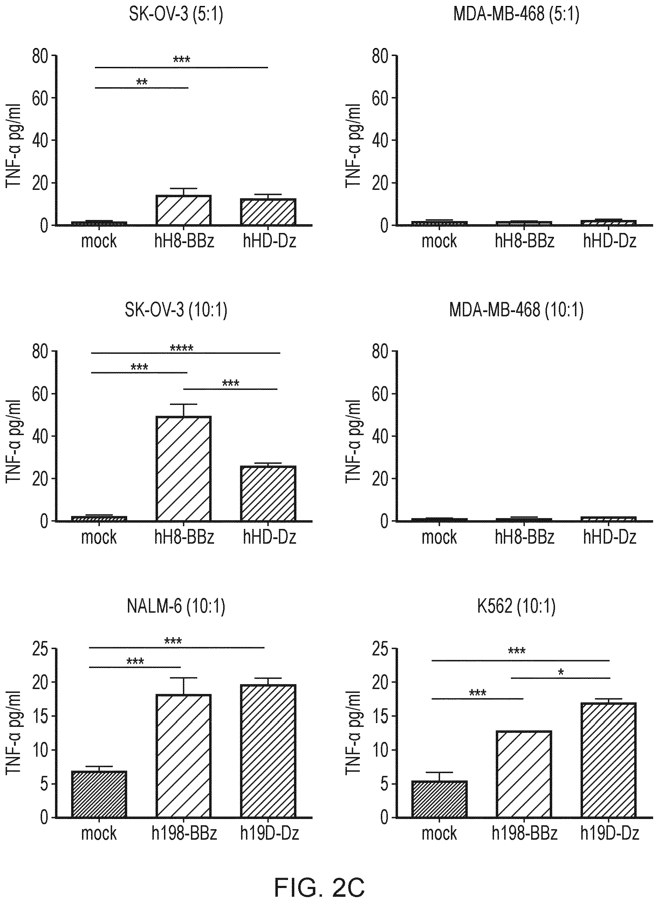

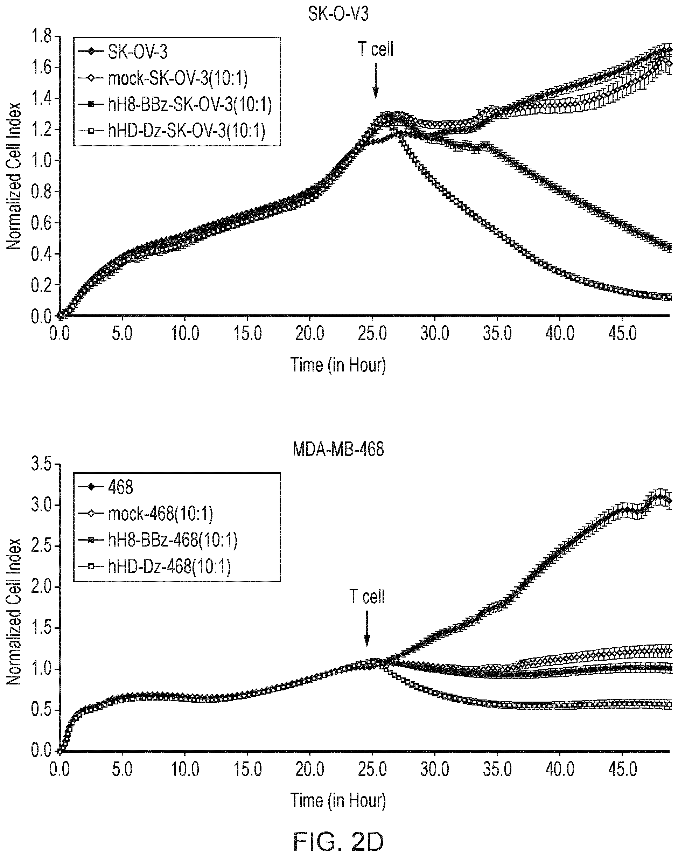

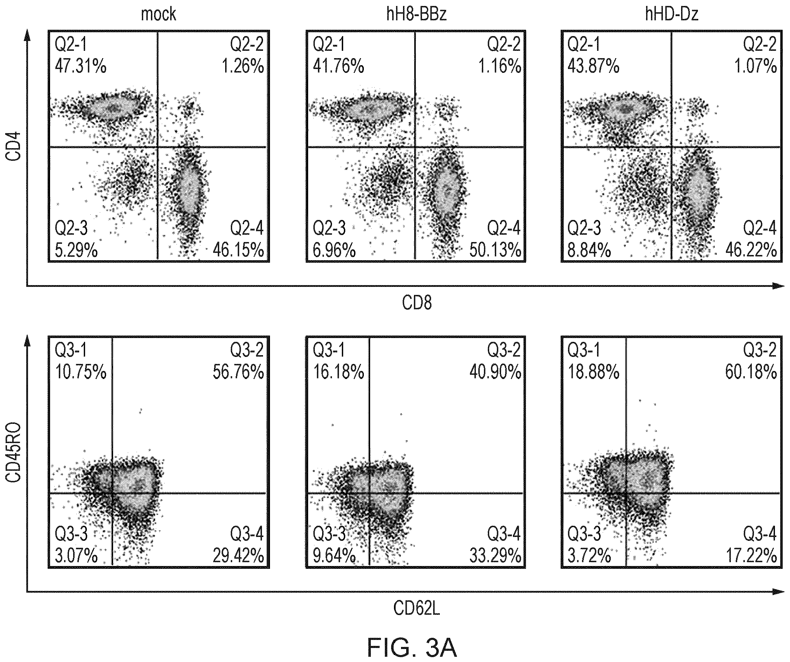

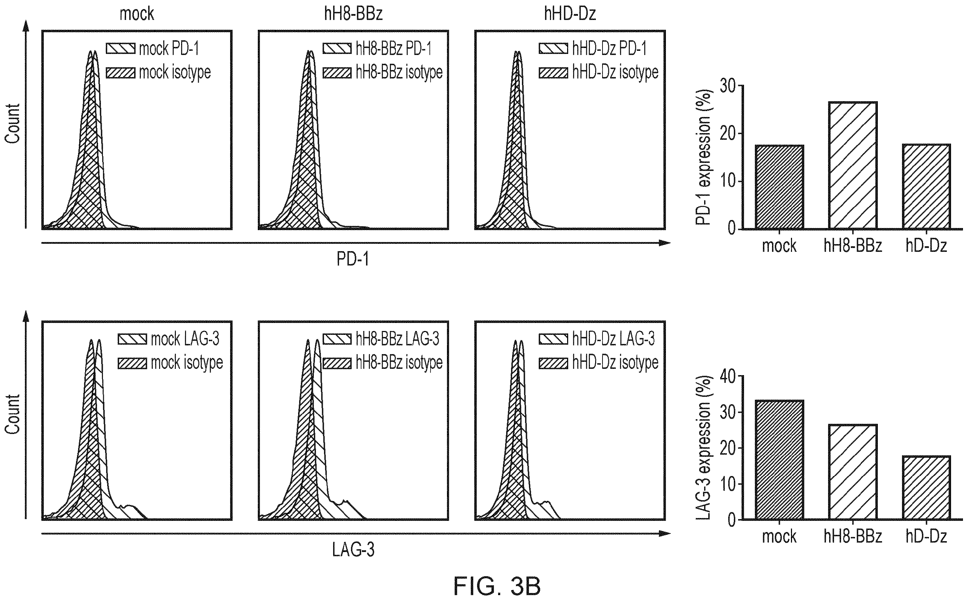

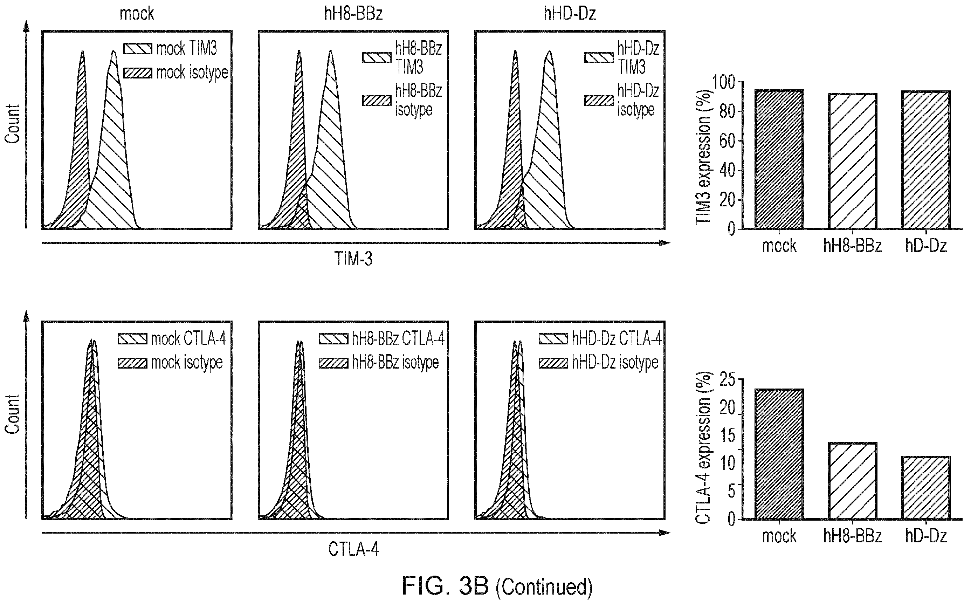

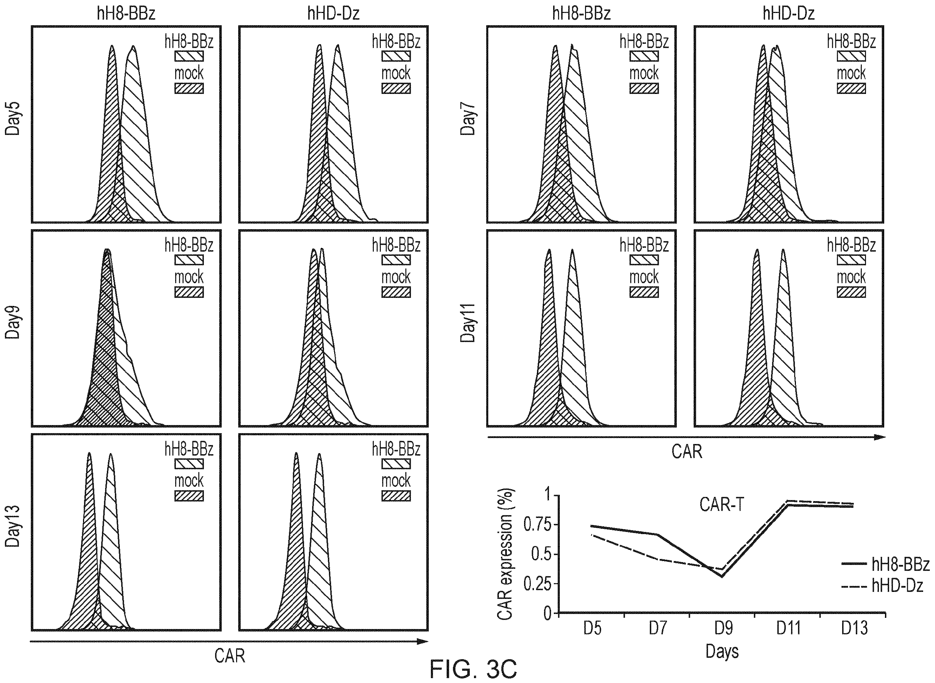

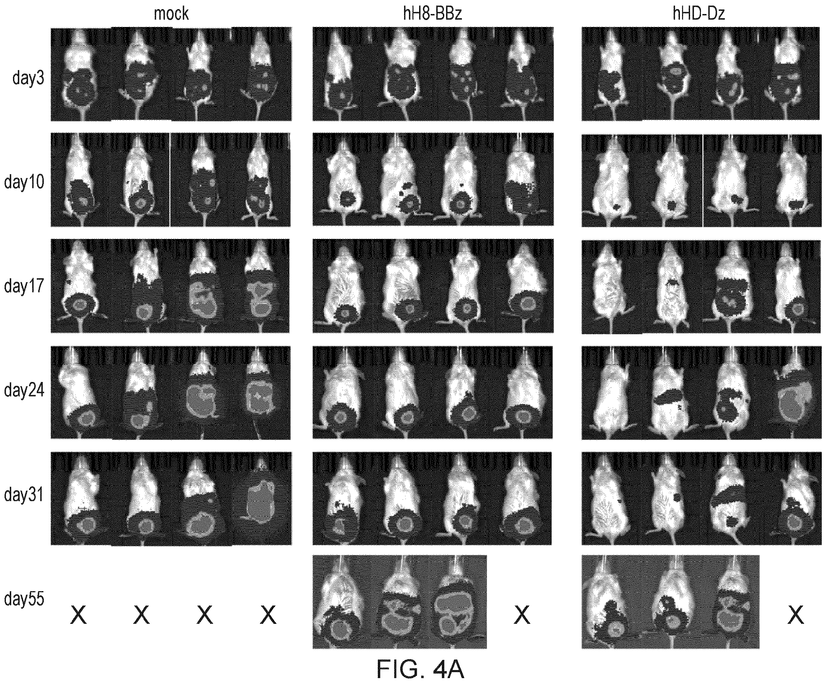

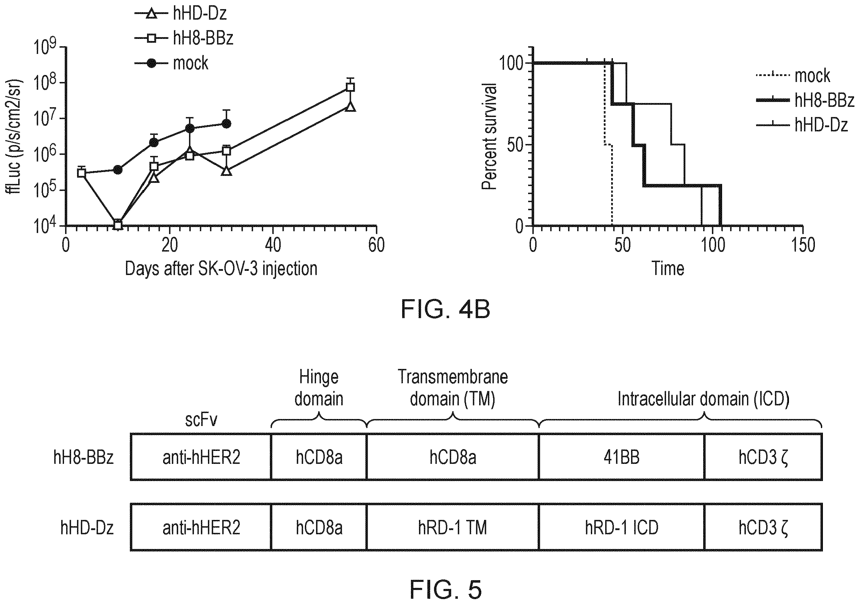

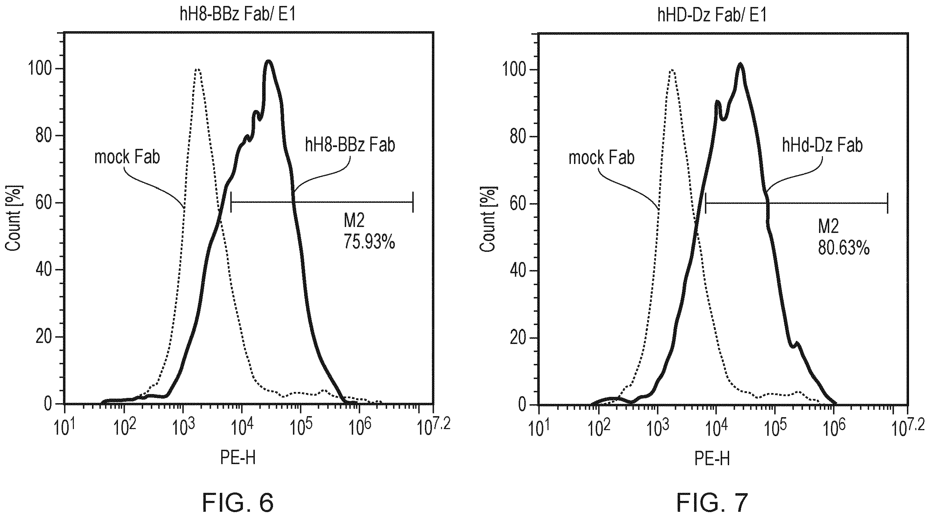

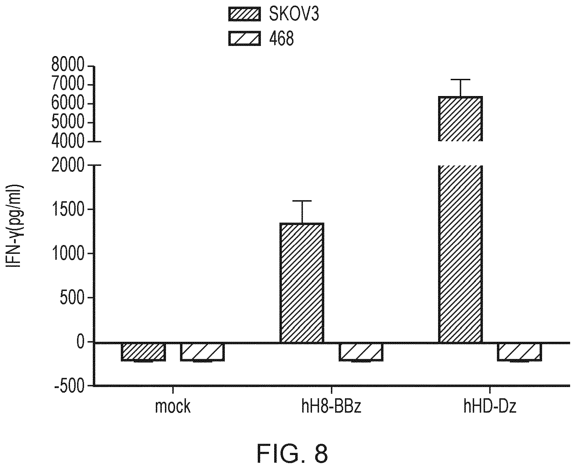

. To make the embodiments of the present invention or the technical solutions in the prior art clearer, the drawings required to be used in the description of the embodiments or the prior art will be briefly introduced below. It is obvious that the drawings described below are some embodiments of the present invention, and that other drawings can be obtained from these drawings for those of ordinary skill in the art without making inventive effort.
. Fig. 1 shows CAR constructs and expression: (A) Schematic representation of chimeric receptors that comprise the single-chain fragment that binds to HER2 or CD19 and differ in the transmembrane and intracellular domains; (B) Surface expression of the hH8-BBz CAR and the hHD-Dz CAR, the h198-BBz CAR and the h19D-Dz CAR on the human T cells;
. Fig. 2 shows in vitro cytokine production and cytotoxicity of the CAR-T cells, (A) IFN-γ, (B) IL-6 and (C) TNF-α quantified by ELISA in supernatants from four different CAR-T cells or mock T cells co-cultured with target positive or negative tumor cells overnight (E:T = 10:1 or 5:1) (n = 3/group); statistical analysis is performed by one-way ANOVA, followed by Tukey's posttest analysis; significance is considered P < 0.05; (D) RTCA analysis shows T cell lytic capacity (n = 4/group);
. Fig. 3 shows phenotype, exhaustion marker expression and CAR expression of the HER2 specific CAR-T cells: (A) Flow cytometer density plots of phenotypic profile of each CAR-T cell: cells with either CD3, CD4, or CD8, or a naive (TN) (CD45RA-/CD62L-) central memory T cells (T_{CM}) (CD45RO+/CD62L+) or effector memory (T_{EM}) (CD45RO+/CD62L-); (B) Flow cytometer density plots of inhibitory molecules of each CAR-T cell: cells with either PD-1, or LAG3, or CTLA-4, or TIM3; (C) The CAR expression of the hH8-BBz CAR-T cells and the hHD-Dz CAR-T cells with time measured by flow cytometry;
. Fig. 4 shows in vivo antitumor activity of the HER2 specific CAR-T cells: (A) In vivo live imaging of the SK-OV-3-luc tumor bearing model (n = 4/group); (B) Kaplan-Meier analysis of the overall survival rate for each group;
. Fig. 5 is a structural schematic diagram of a 2nd generation chimeric antigen receptor (RD-1) targeting to HER2;
. Fig. 6 is an expression assay diagram of human T cells transfected by conventional 2nd generation CAR genes;
. Fig. 7 is an expression assay diagram of human T cells transfected by novel 2nd generation CAR (RD-1) genes; and
. Fig. 8 is an assay diagram of IFN-γ secretion of CAR-T cells (RD-1) targeting to HER2 positive tumor cells SKOV3 and HER2 negative tumor cells MDA-MB-468.

### Detailed Description

. To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

. The embodiments are for the purpose of better illustration of the present invention, but the contents of the present invention are not limited to the embodiments. Therefore, non-essential improvements and adjustments of the embodiments made by those skilled in the art according to the above-mentioned contents of the present invention still belong to the protection scope of the present invention.

. It should be noted that the term "include", "comprise" or any variant thereof is intended to encompass nonexclusive inclusion so that a process, method, article or device including a series of elements includes not only those elements but also other elements which have been not listed definitely or an element(s) inherent to the process, method, article or device. Moreover, the expression "comprising a(n) ... " in which an element is defined will not preclude presence of an additional identical element(s) in a process, method, article or device comprising the defined element(s) unless further defined.

. As used herein, the term "about", typically means +/-5% of the stated value, more typically +/-4% of the stated value, more typically +/-3% of the stated value, more typically, +/-2% of the stated value, even more typically +/-1% of the stated value, and even more typically +/-0.5% of the stated value.

. Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

. "RD-1" is a reverse dectin-1, with an amino acid sequence of "GLVVAIVLIVLCLIGLIVAILRWPPSAACSGKESVVAIRTNSQSDFHLQTYGDE DLNELDPHYEM", as shown in SEQ ID NO:3; whereas an amino acid sequence of a dectin-1 is "MEYHPDLENLDEDGYTQLHFDSQSNTRTAVVSEKGSCAASPPWRLIAVILGI LCLVILVIAVVLG", as shown in SEQ ID NO:4. An amino acid sequence of an "RD-1 intracellular domain" is "RWPPSAACSGKESVVAIRTNSQSDFHLQTYGDEDLNELDPHYEM", as shown in SEQ ID NO:1; and an amino acid sequence of an "RD-1 transmembrane domain" is "GLVVAIVLIVLCLIGLIVAIL", as shown in SEQ ID NO:2. The inventor investigated that in preparation of a novel 2nd generation CAR, using the reverse dectin-1 as a costimulatory domain can efficiently produce and maintain activity and functions of the dectin-1 (i.e. can influence functions of T cells, enhance secretion and lytic capacity of a variety of cytokines (including IFN-γ, TNF-α and IL-6), reduce exhaustion potential, and increase cell expansion and significant antitumor activity). Therefore, unless otherwise specified herein, a dectin-1 in a CAR structure refers to a reverse dectin-1.

### Embodiment 1

### Materials and methods

.
1. Cell lines: all cell lines were obtained from the American Type Culture Collection (ATCC). The K562 (myelogenous leukemia) and NALM6 (lymphoblastic leukemia) were cultured in RPMI-1640 with heat-inactivated 10% fetal bovine serum (FBS) (PAN, Germany. Cat: ST30-3302), penicillin (Gibco, Thermo Fisher, Waltham, MA. Cat: SV30010) (100 U/mL) and streptomycin (Gibco, Thermo Fisher, Waltham, MA. Cat: SV30010) (100 ug/mL). The human cancer cell lines SK-OV-3 (ovarian cystadenocarcinoma) and MDA-MB-468 (breast cancer) were cultured in DMEM with heat-inactivated 10% FBS, penicillin (100 U/mL) and streptomycin (100 ug/mL). SK-OV-3 cells were also engineered to express luciferase.
2. Construction of plasmids encoding CARs
   Anti-CD 19 or anti-HER2 CARs include a single-chain fragment variable (scFv) specific to CD19 (clone FMC63) or HER2 (clone 4D5). The scFv was followed by a human CD8α hinge region, then either a human CD8α transmembrane domain (TM), 4-1BB or CD3ζ intracellular domains (ICDs). CAR sequences are formed by overlapping PCR. Individual lentiviral plasmids encoding each CAR sequence was constructed using double enzymes digestion with the PCLK lentiviral vector. Four CAR sequences constructed were as follows:
   1) hH8-BBZ: HER2 scFv-CD8α(hinge + TM)-4-1BB-CD3ζ ICDs
   2) hHD-DZ: HER2 scFv-CD8α hinge +Dectin-1(TM + cytoplasm) -CD3ζ ICDs
   3) h198-BBZ: CD19 scFv-CD8α(hinge + TM)-4-1BB-CD3ζ ICDs
   4) h19D-DZ: CD19 scFv-CD8α hinge +Dectin-1(TM + cytoplasm) -CD3ζ ICDs
3. Generation of lentiviral particles
   HEK-293T cells (embryonic kidney cells) from ATCC were cultured in DMEM with heat-inactivated 10% fetal bovine serum (FBS) (PAN, Germany. Cat: ST30-3302), penicillin (100 U/mL) and streptomycin (Gibco, Thermo Fisher, Waltham, MA. Cat: SV30010) (100 ug/mL).

. To produce lentivirus-comprising supernatant, HEK-293T cells were transfected with the following plasmids as detailed previously: the appropriate CAR-encoding plasmids, psPAX2 and pMD2.0G (Invitrogen). The medium was changed 12 h after transfection. The supernatant was harvested and spun to get rid of cell debris. The supernatant was filtered and concentrated by ultracentrifugation at 19,700 rpm for 2 h. The supernatant was discarded. The lentiviral pellet was dissolved in PBS medium and the concentrated lentivirus was stored at -80°C. The concentrated lentivirus titers were measured by quantitative real time polymerase chain reaction (Q-RT-PCR).

### 4. Isolation, transduction, production and expansion of CAR-T cells

. Peripheral Blood Mononuclear Cells (PBMCs) were isolated from healthy donor blood by the Ficoll-hypaque density gradient (Lonza, Cat:04-418Q). All samples were obtained after informed consent and approval by the Ethics Committee of the State Key Laboratory of Biotherapy.

. PBMCs were cultured in X-VIVO 15 medium (Sigma-Aldrich, Cat:10771) with 5% human serum (Sigma-Aldrich, H4522) and 100 U/ml recombinant human IL-2 (rhIL-2) (PeproTech, NJ, USA. Cat: 200-02-10). PBMCs were stimulated with anti-CD3/CD28 magnetic beads (Gibco, Thermo Fisher, Waltham, MA. Cat: 11131D). After 24 h, T cells were cultured with the lentivirus at a multiplicity of infection (MOI) of 5 for 48 h, and then the cells were washed and cultured in the T cell medium. Transduction efficiency was determined by CAR expression measured by a flow cytometry.

### 5. Flow cytometry assays

. All flow cytometry assays were performed on a Novocyte flow cytometry (ACEA Biosicences, Inc.), and data were analyzed by novocyte express (ACEA Biosicences, Inc.).

. Transduction efficiency and associated CAR protein expression were evaluated using biotin-SP-conjugated AffiniPure Goat Anti-Mouse IgG, F(ab')2 Fragment Specific (Cat: 120962, Jackson Immune Research) with PE-streptavidin (Cat: 405203, BD Biosciences).

. The following antibodies were used for differentiation phenotype, and exhaustion marker assays:
. anti-CD3-FITC (clone: HIT3a, Cat: 300306, Biolegend), anti-CD8-APC (clone: HIT8a, Cat: 300912, Biolegend), anti-CD4-PE (clone: RPA-T4, Cat: 300508, Biolegend), anti-CD45ROPE (BD Biosciences, clone: UCHL1, Cat: 555493), antiCD62L-APC (BD Biosciences, clone: DREG-56, Cat: 559772), anti-PD-1-APC (clone: EH12.2H7, Cat: 329908, Biolegend), anti-CTLA-4-APC (Cat: 369612, Biolegend), anti-LAG3-APC (Cat: 369212, Biolegend), and anti-TIM3- APC (Cat: 345012, Biolegend).

### 6. In vitro cytokine experiments

. Target cells (NALM6, K562, SK-OV-3 or MDA-MB-468, at 1×10⁵ cells/well) were seeded in 96-well plates and incubated at 37°C with 5% CO₂ overnight. After that, CAR-T cells were added at an effector/target ratio (E:T) of 5 or 10. The CAR-T cell number was normalized by transduction efficiency. Supernatants were collected 24 h after co-culture with the target cells. ELISA kits for cytokine assay from Invitrogen (IFN-γ Cat: 88-7316-88, TNF-α Cat: 88-7346-88, and IL-6 Cat: 88-7066-88) were used to quantify IFN-γ, TNF-α and IL-6.

### 7. Real-time cytotoxicity assays (RTCA)

. The cytotoxic effect of CAR-T cells was measured by the real-time cytotoxicity assay (ACEA Bioscience, Inc. xCELLigence RTCA SP). SK-OV-3 or MDA-MB-468 cells at 1×10⁴ cells/well were cultured in an E-plate 96 for -24 h. CAR-T cells (hH8-BBz and hHD-Dz) or mock T cells were added to the plates at an E:T ratio of 10. Data were acquired and analyzed according to the protocols specified by the manufacturers (ACEA 162 Bioscience, Inc. RTCA Software 2.1).

### 8. In vivo xenograft studies

. Six-week old female B-NSG (NOD-Prkdcscid IL2rgtm1/Bcgen) mice were used in this study. Each mouse received an i.p. injection of 2×10⁶ SK-OV-3-luc cells.

. The SK-OV-3-luc cells were obtained by engineering design of SK-OV-3 cells, which can express luciferase and display tumor growth through fluorescence signals in a subsequent tumor bioluminescence imaging experiment.

. Tumors were allowed to grow for 3 days, and then each mouse received an i.p. injection of 1×10⁷ CAR-T cells (hH8-BBz, hHD-Dz or mock). After 3 additional days, another i.p. injection of the CAR-T cells was given to individual mouse. Bioluminescent imaging (BLI) for tumors was performed on scheduled days (day 3, day 10, day 17, day 24, day 31 and day 55) by IVIS (in vivo imaging system). Tumor fluxes (photon/s/cm²/steradian) were quantified by measuring the photon signal within a delineated region of interest (ROI) encompassing. Living Image software (v2.50, Xenogen; Caliper Life Sciences) was used to demonstrate the BLI data. The data for survival analysis was established at the death of each mouse.

### 9. Statistics

. Statistical plotting and analysis were performed using GraphPad Prism v6.01 (GraphPad Software Inc.) and SPSS V17. Data were expressed as the mean ± SD. One-way ANOVA was used for comparison of three groups in a single condition.

. Kaplan-Meier survival data were analyzed using a log rank (Mantel-Cox) test. Data were transformed when needed to normalize variance. Symbols indicate statistical significance as follows: ^{∗} *P* < 0.05; ^{∗∗} *P* < 0.01 and ^{∗∗∗} *P* < 0.001.

### Embodiment 2

### 1. Novel CAR constructs with the dectin-1 costimulatory signaling domain

. In the present invention, novel 2nd generation CAR constructs were designed through the combination of scFv domains targeting either CD19 or HER2 epitope with the dectin-1 TM, dectin-1 and CD3ζ ICDs (Fig. 1A). Two more CAR constructs were generated with the components of the most popular 2nd generation CAR structure (human CD8α TM with 4-1BB and CD3ζ ICDs). All CAR constructs were well expressed on the surface of T cells (Fig. 1B). As to the anti-HER2 CARs, the hH8-BBz (4-1BB) CAR expression ratio was 49.61%, and the hHD-Dz (dectin-1) CAR expression ratio was 46.17%. As to the anti-CD19 CARs, the h198-BBz (4-1BB) CAR expression ratio was 92.95%, and the h19D-Dz (dectin-1) CAR expression ratio was 95.07%. Although the expression of anti-HER2 CARs was lower than anti-CD19 CARs, there was no clear difference in the expression level of anti-HER2 or anti-CD19 CARs comprising different costimulatory signaling domains.

### 2. Impacts on effector functions of novel CAR-T cells through dectin-1 costimulation

. Typically, a later effector function by CAR-T cells can be assessed by cytokine secretion. Therefore, the impacts of dectin-1 as a costimulatory signaling molecule on cytokine release from the new CAR-T cells were evaluated following exposure to tumor cells that express either HER2 or CD19.

. The released effector cytokines, including IFN-γ, TNF-α and IL-6, by anti-HER2 or anti-CD19 CAR-T cells increased significantly with incorporating the dectin-1 signaling domain in the target positive cell lines (SK-OV-3 and NALM6) (Fig. 2A, 2B and 2C). Anti-HER2 CAR-T cells in this study showed no significant increase of cytokine production against MD-MB-468 (negative cell lines) (Fig. 2A, 2B and 2C). In SK-OV-3 cell line, the hHD-Dz CAR-T cells showed higher levels of IFN-γ than hH8-BBz CAR-T cells; in contrast, the hH8-BBz CAR-T cells secreted more TNF-α (Fig. 2A, 2B and 2C). In NALM6 cell line, the h19D-Dz CAR-T cells produced similar levels of IFN-γ and TNF-α to the h198-BBz CART cells (Fig. 2A, 2B and 2C).

. The cytotoxic function of anti-HER2 CAR-T cells was further investigated to illustrate antigen engagement and CAR-T cell activation. After co-culture of anti-HER2 CAR-T cells with either SK-OV-3 or MDA-MB-468 tumor cell lines, it was observed that both hHD-Dz and hH8-BBz CAR-T cells effectively lysed the SK-OV-3 tumor cells, and hHD-Dz CAR-T cells showed a much different lytic cytotoxicity function from the hH8-BBz CAR-T cells (Fig. 2D).

### 3. Phenotype and exhaustion marker expression of anti-HER2 CAR-T cells

. The phenotype and exhaustion marker expression of both anti-HER2 CAR-T cells and mock T cells were analyzed after a 7-day period of cell expansion (Fig. 3).

. The hHD-Dz, hH8-BBz CAR-T and mock T cells showed similar percentage of CD4⁺ or CD8⁺ T cells (Fig. 3A). Although comparable percentage of effector memory T (T_{EM}, CD45RO+ CD62L-) was observed in the hHD-Dz and hH8-BBz CAR-T cells, the hHD-Dz CAR-T cells showed a higher percentage of central memory T cells (T_{CM}, CD45RO+ CD62L+) than the hH8-BBz CAR-T cells (Fig. 3A).

. The expression pattern of inhibitory receptors, including PD-1, CTLA-4, TIM3 and LAG3, was assessed for the anti-HER2 CAR-T cells (Fig. 3B) in the present invention.

. It was showed that there were -10% less PD-1 or LAG3 positive cells in the hHD-Dz CAR-T cells than in the hH8-BBz CAR-T cells. In terms of TIM3 and CTLA-4 positive cells, a similar percentage was observed in the hHD-Dz and the hH8-BBz CAR-T cells (Fig. 3B).

. The time-course of anti-HER2 CAR expression was further explored in the present invention. Although there was a decrease of CAR expression within 96 h, the CAR expression reached to >90% after additional 48 h (Fig. 3C).

. Overall, the above results suggested that the dectin-1 signaling domain in the novel CAR-T cells may result in distinct phenotype and exhaustion marker expression, and discrete T cell proliferation potential.

### 4. In vivo antitumor activity of anti-HER2 CAR-T cells

. Using NSG mice bearing xenograft SK-OV-3-LUc tumor cells, in vivo antitumor activity of the anti-HER2 CAR-T cells (the hH8-BBz or hHD-Dz CAR-T cells) was further investigated in the present invention. Overall survival and tumor volume were evaluated (Fig. 4). Treatment with the anti-HER2 CAR-T cells resulted in a delayed tumor progression compared to the mock T cell treated mice (Fig. 4A), and the anti-HER2 CAR-T cell groups at least doubled the median survival of tumor bearing mice (Fig. 4B). The log rank (Mantel-Cox) test demonstrated a statistically significant difference in the survival rates between either anti-HER2 CAR-T cell group and mock T cell group. Moreover, 100% of mice in the hHD-Dz CAR-T cell group were alive by day 55, and the hH8-BBz CAR-T group showed longer overall survival (Fig. 4B).

### Embodiment 3

. The embodiment further provides a costimulatory signaling domain of a chimeric antigen receptor, wherein the costimulatory signaling domain comprises an RD-1 intracellular domain.

. Further, the costimulatory signaling domain comprises CD3ζ.

. Further, the RD-1 intracellular domain comprises an amino acid sequence of SEQ ID NO:1 or an amino acid sequence having not less than 90% identity with SEQ ID NO:1.

. Further, the costimulatory signaling domain further comprises one or more of CD3, CD4, CD8, FcR, DAP10, DAP12, CD27, CD28, CD137, CD134, ICOS, OX40, CD30, CD40, PD-1, LFA-1, CD2, CD7, LIGHI, NKG2C, B7-H3, CD83-specific binding ligand, CDS, ICAM-1, GITR, BAFFR, HVEM (IGHTR), SLAMF7, NKp80 KLRE1, CD160, CD19, CD83, IL-2Rβ, IL-Rγ, IL-7Rα, ITGA4, VLA1, CD49α, ITGA4, IA4, CD49D, ITGA6, LA-6, CD49f, ITGAD, CD11d, ITGAE, CD103, ITGAL, CD11α, LFA-1, ITGAM, CDIIB, ITGAX, CD11c, ITGB1, CD29, ITGB2, CD18, LFA-1, ITGB7 TAFR2, TRANCE/RANKL, DNAM1 (CD226), SLAMF4 (CD244, 2B4), CD84, CD96(Tactile), CEACAM1 CRTAM, Ly9 (CD229), D160 (BY55), PSGL1, CD100 (SEMA4D), CD69, SLAMF6 (NTB-A, Ly108 SLAM (SLAMF1, CD150, IPO-3), BLAME (SLAME8), SELPLG (CD162), LTBR, LAT, GADS, SLP-76 PAG/Cbp, NKp4, NWKp30, NKp46 and NKG2D.

. The embodiment further provides a chimeric antigen receptor comprising the above-mentioned costimulatory signaling domain.

. Further, the chimeric antigen receptor comprises an antigen recognition domain, a CD8α hinge region, a transmembrane domain, an RD-1 intracellular domain and CD3ζ which are connected in sequence.

. Further, an amino acid sequence comprised in the transmembrane domain is capable of anchoring the chimeric antigen receptor to a cell membrane.

. Further, the transmembrane domain comprises an RD-1 transmembrane domain.

. Further, the RD-1 transmembrane domain comprises an amino acid sequence of SEQ ID NO:2 or an amino acid sequence having not less than 90% identity with SEQ ID NO:2.

. Preferably, the antigen recognition domain is a HER2 binding domain.

. Further, the chimeric antigen receptor comprises an amino acid sequence of SEQ ID NO:5.

. Preferably, the antigen recognition domain is a CD19 binding domain.

. Further, the chimeric antigen receptor comprises an amino acid sequence of SEQ ID NO:6.

. The embodiment further provides a synthesis method of the above-mentioned chimeric antigen receptor, wherein the method comprises the following steps: step (1) synthesizing a gene sequence of RD-1-CD3ζ; step (2) synthesizing a gene sequence of the chimeric antigen receptor.

. Further, the step (2) specifically comprises: first using primers F1 and R1 to expand a bound gene sequence of the antigen recognition domain and the CD8α hinge region, then using primers F2 and R2 to expand the gene sequence of RD-1-CD3ζ, and finally using the bound gene sequence of the antigen recognition domain and the CD8α hinge region as well as the gene sequence of RD-1-CD3ζ as templates and using F1 and R2 as primers to synthesize the gene sequence of the chimeric antigen receptor.

. Further, the primer F1 is shown in SEQ ID NO:7, R1 is shown in SEQ ID NO:8, F2 is shown in SEQ ID NO:9, and R2 is shown in SEQ ID NO:10.

. An amino acid sequence of the synthesized (anti-HER2 scFV)-(CD8α hinge)-(RD-1TM+Cytoplasmic)-(CD3ζ) is SEQ ID NO:5.

. The embodiment further provides a recombinant plasmid vector constructed by the above-mentioned chimeric antigen receptor and an expression vector.

. Further, the expression vector is a pCLK vector.

. The embodiment further provides a construction method of the above-mentioned recombinant plasmid vector, wherein the construction method comprises: using Mlu I and Spe I as restriction sites to connect genes of the chimeric antigen receptor with the expression vector, and thus to obtain the recombinant plasmid vector.

. The embodiment further provides an immune cell modified by the above-mentioned chimeric antigen receptor.

. Further, the immune cell is T lymphocyte.

. Further, the immune cell also includes one or more of B lymphocyte, K lymphocyte and NK lymphocyte.

. The embodiment further provides a method for obtaining the above-mentioned immune cell, wherein the method comprises the following steps: using Mlu I and Spe I as restriction sites to connect genes of the chimeric antigen receptor with an expression vector, and thus to obtain a recombinant plasmid vector; transfecting the recombinant plasmid vector and packaging plasmids together into cultured cells for culturing, thus to obtain recombinant virus particles; and using the recombinant virus particles to modify the immune cell.

. Further, method of the modification comprises: using a virus vector system or non-virus vector system.

. Further, the virus vector system includes one or more of a retroviral vector, a lentiviral vector, an adenovirus vector, an adeno-associated virus vector and a sendai virus vector.

. Further, the non-virus vector system includes one or more of a transposon system, a CRISPR gene editing system, a TALEN system, a liposome transfection system and an electrotransfection system.

. The purpose of the present invention is to further provide an application of the above-mentioned immune cell in preparation of an antitumor drug composition.

. Further, the antitumor drug composition further comprises a chemical drug.

. Further, the chemical drug includes cyclophosphamide and/or fludarabine.

. Further, the antitumor drug composition is an antitumor drug against breast cancer and/or ovarian cancer.

. The embodiment further provides a composition comprising the above-mentioned chimeric antigen receptor, wherein the composition further comprises one or more of a β-glucan receptor, Syk, CR3 and CD11b bound to the costimulatory signaling domain.

. The primers used for obtaining CAR genes in the embodiment are shown in Table 1:

.

**Table 1 Primers used for obtaining CAR genes**

| Primer | Sequence ID Number | Sequence |
|---|---|---|
| F1 | SEQ ID NO:7 | CGACGCGTatggccttaccagtga |
| R1 | SEQ ID NO:8 | TGCACACGAGGGGGCTGGACTTCGCCTGTGATggtctggtc gtggctatagtgctgatagtatt |
| F2 | SEQ ID NO:9 | aatactatcagcactatagccacgaccagaccATCACAGGCGAAGTCCA GCCCCCTCGTGTGCA |
| R2 | SEQ ID NO:10 | GGACTAGTttaGCGAGGGGG |

### Embodiment 4

### Obtaining CAR genes with a full-length antigen of HER2 by PCR:

(1) Step 1, gene synthesis: synthesizing a full length of an RD-1 (TM+cytoplasmic)-CD3ζ fragment.
(2) Step 2, synthesizing primers, and obtaining a fused fragment of (anti-HER2 scFV)-(CD8α hinge)-(RD-1TM+Cytoplasmic)-(CD3ζ) by an overlapping PCR method, which specifically comprises the following two steps:
   (2.1) Expanding (anti-HER2 scFV)-(CD8α hinge) using primers F1 and R1; and expanding (RD-1TM+Cytoplasmic)-(CD3ζ) using primers F2 and R2.
   (2.2) Using (anti-HER2 scFV)-(CD8α hinge) and (RD-1TM+Cytoplasmic)-(CD3ζ) as templates and using F1 and R2 as primers to obtain full-length CAR genes by PCR.

. An amino acid sequence of the synthesized (anti-HER2 scFV)-(CD8α hinge)-(RD-1TM+Cytoplasmic)-(CD3ζ) is SEQ ID NO:5.

### . Construction of a recombinant plasmid vector for CAR with an antigen of HER2:

(1) Step 1, gene synthesis: synthesizing a full length of an RD-1 (TM+cytoplasmic)-CD3ζ fragment.
(2) Step 2, designing primers, and obtaining a fused fragment of (anti-HER2 scFV)-(CD8α hinge)-(RD-1TM+Cytoplasmic)-(CD3ζ) by an overlapping PCR method, which specifically comprises the following two steps:
   (2.1) Expanding (anti-HER2 scFV)-(CD8α hinge) using primers F1 and R1; and expanding (RD-1TM+Cytoplasmic)-(CD3ζ) using primers F2 and R2;
   (2.2) Using (anti-HER2 scFV)-(CD8α hinge) and (RD-1TM+Cytoplasmic)-(CD3ζ) as templates and using F1 and R2 as primers to obtain full-length CAR genes by PCR.
(3) Step 3, using Mlu I and Spe I as restriction sites to connect the CAR genes synthesized by the above steps with a PCLK vector, and thus to obtain a CAR-PCLK plasmid vector.

### . Virus transfection of T lymphocyte and expression assay of target genes:

(1) Loading novel 2nd generation CAR genes (anti-HER2 scFV)-(CD8α hinge)-(RD-1TM+Cytoplasmic)-(CD3ζ) with chimeric antigen receptor genes (the structural schematic diagram is shown in Fig. 5) and conventional 2nd generation CAR genes (anti-HER2 scFV)-(CD8α hinge+TM)-(4-1BB)-(CD3ζ) (the structural schematic diagram is shown in Fig. 5) into the lentiviral vector PCLK which is co-transfected into T cells together with two helper vectors psPAX2 and pMD2.G, resulting in respective packaged virus particles which is concentrated to obtain high-concentration lentiviral vector through centrifugation.
(2) Isolating lymphocytes by density gradient centrifugation, and stimulating the lymphocytes with a CD3 antibody (1 ug/ml) and IL-2 (100 IU/ml). After one day, collecting the lymphocytes to perform virus transfection, culturing the lymphocytes for 48 h, and collecting the transfected lymphocytes.
(3) Using a specific antibody which recognizes antibody Fab fragments to perform a flow cytometry assay on the collected virus-transfected lymphocytes.

. Fig. 6 is an expression assay of a human T cell transfected by the conventional 2nd generation CAR genes, wherein the primary antibody used is a biotin-labeled goat anti-mouse Fab monoclonal antibody, and the secondary antibody used is a PE-labeled anti-streptavidin antibody (for flow cytometry). The first lane is a T cell transfected with empty virus, and the second lane is a T cell transfected with CAR genes. (In Fig. 2, hH8-BBz represents the conventional 2nd generation CAR genes (anti-HER2 scFV)-(CD8α hinge+TM)-(4-1BB)-(CD3ζ)).

. Fig. 7 is an expression assay of a human T cell transfected by the novel 2nd generation CAR genes, wherein the primary antibody used is a biotin-labeled goat anti-mouse Fab monoclonal antibody, and the secondary antibody used is a PE-labeled anti-streptavidin antibody (for flow cytometry). The first lane is a T cell transfected with empty virus, and the second lane is a T cell transfected with CAR genes. (In Fig. 3, hHD-Dz represents the novel 2nd generation CAR genes (anti-HER2 scFV)-(CD8α hinge)-(RD-1TM+Cytoplasmic)-(CD3ζ)).

. It is confirmed by flow cytometry assays that both novel and conventional 2nd generation CAR molecules are efficiently expressed on surface of lymphocytes under the same MOI (multiplicity of infection) virus transfection effect.

### . Killing effect on tumor cells expressing HER2:

Conventional 2nd generation CAR-T cells: (anti-HER2 scFV)-(CD8α hinge+TM)-(4-1BB)-(CD3ζ)-T cells
Novel 2nd generation CAR-T cells: (anti-HER2 scFV)-(CD8α hinge)-(RD-1TM+Cytoplasmic)-(CD3ζ)-T cells
   (1) Respectively measuring the killing effect of the two kinds of T cells on HER2 positive tumor cells SKOV3 (human ovarian cancer cells) by RTCA (RealTime Cellular Analysis), and the measurement results are shown in Fig. 2D. (In Fig. 2D, hH8-BBz represents the conventional 2nd generation CAR-T cells; and hHD-Dz represents the novel 2nd generation CAR-T cells).
   (2) Respectively measuring cytokine secretion of the two kinds of CAR-T cells targeting to HER2 positive tumor cells SKOV3 (human ovarian cancer cells) and HER2 negative tumor cells MDA-MB-468 (human breast cancer cells) by ELISA, and the measurement analysis results are shown in Fig. 8. Fig. 8 clearly shows that more cytokine (IFNγ) is secreted to the HER2 positive tumor cells SKOV3 by the novel 2nd generation CAR-T cells than by the conventional 2nd generation CAR-T cells. (In Fig. 8, hH8-BBz represents the conventional 2nd generation CAR genes (anti-HER2 scFV)-(CD8α hinge+TM)-(4-1BB)-(CD3ζ); and hHD-Dz represents the novel 2nd generation CAR genes (anti-HER2 scFV)-(CD8α hinge)-(RD-1TM+Cytoplasmic)-(CD3ζ)).

### . Conclusion

. A typical CAR mainly consists of three key components, including a single-chain fragment variable (scFv) to recognize antigen, a hinge and transmembrane domain (TM), such as CD3, CD28 or CD8 protein, and intracellular signaling domains (ICDs), such as CD3ζ or FcRy. The CAR includes one or more intracellular costimulatory signaling domains, such as CD28, 4-1BB, CD27, OX40, ICOS, DAP10, IL-15Rα, MyD88/CD40 and TLR2, to transmit activation signals.

. It has been showed that different TMs and/or ICDs affect T cell expansion, persistence and other functions. Recently, several 2nd generation CARs have been tested in patients with solid tumors, such as metastatic colorectal cancers and sarcomas. The results from these trials were far from exciting compared with that achieved in treating hematological malignancies. Therefore, exploring different costimulatory domains may provide a new approach to improve antitumor effects of CAR-T cells in the solid tumors.

. In the present invention, CD19 or HER2-targeting scFv domains were coupled to 4-1BB or dectin-1 signaling ICDs by our experiment team to construct four different 2nd generation CARs. Experiment data revealed that the novel CAR design influenced T cell functions through dectin-1 costimulation in both in vitro and in vivo experiments, such as enhanced cytokine secretion and lytic capacity, reduced exhaustion potential, and increased cell expansion and distinct antitumor activity.

. In the present invention, we confirmed previous study results showing enhanced CAR-T cell functions with 4-1BB costimulatory signaling domains. Interestingly, in vitro T cell functions (e.g. increased cytokine production) of the hHD-Dz CAR-T cells are comparable to the 4-1BB based, and both are superior to the mock T cells. As to the HER2 specific CAR-T cells, the IFN-γ secretion of the hHD-Dz CAR-T cells was higher than the hH8-BBz, suggesting possible predominantly Th1 phenotype; while the Hh8-bbz CAR-T cells released more TNF-α, consistent with Th1/Th2 phenotype. Similar cytokine production pattern by the anti-CD19 CAR-T cells indicated comparable phenotype irrespective to the specific costimulatory signaling domain employed. In the RTCA (real-time cytotoxicity assays), we illustrated the cytotoxic ability of the hHD-Dz CAR-T cells, superior to the hH8-BBz CAR-T cells. The above results may suggest that the dectin-1 costimulation provides a new mechanistic approach in CAR-T cell immunotherapy in treating solid tumors.

. Due to the immune resistance and T cell exhaustion, one of the biggest challenges in CAR-T cell therapy for solid tumors is the inhibition of tumor microenvironment. However, it has been shown in studies that different T cell phenotype may play an important role in the antitumor immunity, for example, T_{CM} cells are more important than T_{EM} cells in the adoptive immunotherapies. In the present invention, we have found that more T_{CM} and distinct exhaustion marker expression in the hHD-Dz CAR-T cells may suggest that the new CAR-T cells can be less influenced by the tumor immunosuppressive microenvironment through the dectin-1 costimulatory signaling in the solid tumors.

. In addition, we demonstrated the distinct antitumor effects of the 2nd generation CAR-T cells through either dectin-1 or 4-1BB costimulation in the established tumor xenograft model. However, the CARs with the different costimulatory signaling domains here showed discrete antitumor activity trend on in vivo survival rate. The hHD-Dz CAR-T cells showed increased effector functions at early time points, and the hH8-BBz CAR-T cells demonstrated a later antitumor activity. These observations suggested that different costimulatory signaling domains may result in distinct T cell phenotype.

. The embodiments of the present invention are described above in combination with drawings, but the present invention is not limited to the aforementioned specific embodiments. The aforementioned embodiments are merely illustrative and not limiting. For those of ordinary skill in the art, many forms can be made under the teaching of present invention without departing from the spirit of the present invention and the scope of the claims, all of which shall fall within the protection scope of the present invention.

### . Reference

. Kochenderfer JN, Dudley ME, Kassim SH, Somerville RP, Carpenter RO,Stetler-Stevenson M, etal.Chemotherapy-refractory diffuse large B-cell lymphoma and indolent B-cell malignancies can be effectively treated with autologous T cells expressing an anti-CD19 chimeric antigen receptor. Journal of clinical oncology: official journal of the American Society of Clinical Oncology 2015, 33(6): 540-549.
. Chimeric Antigen Receptor-Modified T Cells in Chronic Lymphoid Leukemia; Chimeric Antigen Receptor-Modified T Cells for Acute Lymphoid Leukemia; Chimeric Antigen Receptor T Cells for Sustained Remissions in Leukemia. The New England journal of medicine 2016, 374(10): 998.
. Elsallab M, Levine BL, Wayne AS, Abou-El-Enein M. CAR T-cell product performance in haematological malignancies before and after marketing authorisation. The LancetOncology 2020, 21(2): e104-e116.
. Cohen AD, Garfall AL, Stadtmauer EA, Melenhorst JJ, Lacey SF, Lancaster E, et al. B cell maturation antigen-specific CAR T cells are clinically active in multiple myeloma.The Journal of clinical investigation 2019, 129(6): 2210-2221.
. Raje N, Berdeja J, Lin Y, Siegel D, Jagannath S, Madduri D, et al. Anti-BCMA CART-Cell Therapy bb2121 in Relapsed or Refractory Multiple Myeloma. The NewEngland journal of medicine 2019, 380(18): 1726-1737.
. Yan Z, Cao J, Cheng H, Qiao J, Zhang H, Wang Y, et al. A combination of humanizedanti-CD19 and anti-BCMA CAR T cells in patients with relapsed or refractory multiple myeloma: a single-arm, phase 2 trial. The Lancet Haematology 2019, 6(10):e521-e529.
. Neelapu SS, Locke FL, Bartlett NL, Lekakis LJ, Miklos DB, Jacobson CA, et al.Axicabtagene Ciloleucel CAR T-Cell Therapy in Refractory Large B-Cell Lymphoma.The New England journal of medicine 2017, 377(26): 2531-2544.
. Schuster SJ, Svoboda J, Chong EA, Nasta SD, Mato AR, Anak O, et al. Chimeric Antigen Receptor T Cells in Refractory B-Cell Lymphomas. The New England journal of medicine 2017, 377(26): 2545-2554.
. Locke FL, Ghobadi A, Jacobson CA, Miklos DB, Lekakis LJ, Oluwole OO, et al.Long-term safety and activity of axicabtagene ciloleucel in refractory large B-cell lymphoma (ZUMA-1): a single-arm, multicentre, phase 1-2 trial. The Lancet Oncology2018.
. Locke FL, Ghobadi A, Jacobson CA, Miklos DB, Lekakis LJ, Oluwole OO, et al.Long-term safety and activity of axicabtagene ciloleucel in refractory large B-cell lymphoma (ZUMA-1): a single-arm, multicentre, phase 1-2 trial. The Lancet Oncology2019, 20(1): 31-42.
. Yu S, Li A, Liu Q, Li T, Yuan X, Han X, et al. Chimeric antigen receptor T cells: a novel therapy for solid tumors. Journal of hematology & oncology 2017, 10(1): 78.
. Wang Y, Luo F, Yang J, Zhao C, Chu Y. New Chimeric Antigen Receptor Design for Solid Tumors.Frontiers in immunology 2017, 8: 1934.
. Robbins PF, Dudley ME, Wunderlich J, El-Gamil M, Li YF, Zhou J, et al. Cutting edge: persistence of transferred lymphocyte clonotypes correlates with cancer regression inpatients receiving cell transfer therapy. Journal of immunology 2004, 173(12):7125-7130.
. Ying Z, Huang XF, Xiang X, Liu Y, Kang X, Song Y, et al. A safe and potent anti-CD19CAR T cell therapy.Nature medicine 2019, 25(6): 947-953.
. Long AH, Haso WM, Shern JF, Wanhainen KM, Murgai M, Ingaramo M, et al. 4-1BBcostimulation ameliorates T cell exhaustion induced by tonic signaling of chimeric antigen receptors. Nature medicine 2015, 21(6): 581-590.
. Zimara N, Chanyalew M, Aseffa A, van Zandbergen G, Lepenies B, Schmid M, et al.Dectin-1 Positive Dendritic Cells Expand after Infection with Leishmania major Parasites and Represent Promising Targets for Vaccine Development. Frontiers inimmunology 2018, 9: 263.
. Ariizumi K, Shen GL, Shikano S, Xu S, Ritter R, 3rd, Kumamoto T, et al. Identification of a novel, dendritic cell-associated molecule, dectin-1, by subtractive cDNA cloning.The Journal of biological chemistry 2000, 275(26): 20157-20167.
. Taylor PR, Brown GD, Reid DM, Willment JA, Martinez-Pomares L, Gordon S, et al.The beta-glucan receptor, dectin-1, is predominantly expressed on the surface of cells of the monocyte/macrophage and neutrophil lineages. Journal of immunology 2002,169(7): 3876-3882.
. Adams EL, Rice PJ, Graves B, Ensley HE, Yu H, Brown GD, et al. Differential high-affinity interaction of dectin-1 with natural or synthetic glucans is dependent upon primary structure and is influenced by polymer chain length and side-chain branching.The Journal of pharmacology and experimental therapeutics 2008, 325(1): 115-123.
. Kochenderfer JN, Feldman SA, Zhao Y, Xu H, Black MA, Morgan RA, et al.Construction and preclinical evaluation of an anti-CD19 chimeric antigen receptor.Journal of immunotherapy 2009, 32(7): 689-702.
. Kochenderfer JN, Dudley ME, Feldman SA, Wilson WH, SpanerDE, Maric I, et al.B-cell depletion and remissions of malignancy along with cytokine-associated toxicity in a clinical trial of anti-CD19 chimeric-antigen-receptor-transduced T cells. Blood2012, 119(12): 2709-2720.
. Carter P, Presta L, Gorman CM, Ridgway JB, Henner D, Wong WL, et al.Humanization of an anti-p185HER2 antibody for human cancer therapy. Proceedings of the National Academy of Sciences of the United States of America 1992, 89(10):4285-4289.
. Huang Y, Li D, Zhang PF, Liu M, Liang X, Yang X, et al. IL-18R-dependent and independent pathways account for IL-18-enhanced antitumor ability of CAR-T cells.FASEB journal : official publication of the Federation of American Societies forExperimental Biology 2020, 34(1): 1768-1782.
. Cerignoli F, Abassi YA, Lamarche BJ, Guenther G, Santa Ana D, Guimet D, et al. Invitro immunotherapy potency assays using real-time cell analysis. PloS one 2018,13(3): e0193498.
. Geijtenbeek TB, Gringhuis SI. Signalling through C-type lectin receptors: shaping immune responses. Nature reviews Immunology 2009, 9(7): 465-479.
. Li D, Li X, Zhou WL, Huang Y, Liang X, Jiang L, et al. Genetically engineered T cells for cancer immunotherapy. Signal transduction and targeted therapy 2019, 4: 35.
. Ying Z, He T, Wang X, Zheng W, Lin N, Tu M, et al. Parallel Comparison of 4-1BB orCD28 Co-stimulated CD19-Targeted CAR-T Cells for B Cell Non-Hodgkin's Lymphoma. Molecular therapy oncolytics 2019, 15: 60-68.
. Li S, Tao Z, Xu Y, Liu J, An N, Wang Y, et al. CD33-Specific Chimeric Antigen Receptor T Cells with Different Co-Stimulators Showed Potent Anti-Leukemia Efficacy and Different Phenotype. Human gene therapy 2018, 29(5): 626-639.
. Xia AL, Wang XC, Lu YJ, Lu XJ, Sun B. Chimeric-antigen receptor T (CAR-T) celltherapy for solid tumors: challenges and opportunities. Oncotarget 2017, 8(52):90521-90531.
. Sadelain M, Brentjens R, Riviere I. The basic principles of chimeric antigen receptor design. Cancer discovery 2013, 3(4): 388-398.
. Zolov SN, Rietberg SP, Bonifant CL. Programmed cell death protein 1 activation preferentially inhibits CD28.CAR-T cells. Cytotherapy 2018, 20(10): 1259-1266.
. Li G, Boucher JC, Kotani H, Park K, Zhang Y, Shrestha B, et al. 4-1BB enhancement of CAR T function requires NF-kappaB and TRAFs. JCI insight 2018, 3(18).
. Kowolik CM, Topp MS, Gonzalez S, Pfeiffer T, Olivares S, Gonzalez N, et al. CD28costimulation provided through a CD19-specific chimeric antigen receptor enhances in vivo persistence and antitumor efficacy of adoptively transferred T cells. Cancerresearch 2006, 66(22): 10995-11004.
. Zhang JP, Zhang R, Tsao ST, Liu YC, Chen X, Lu DP, et al. Sequential allogeneic andautologous CAR-T-cell therapy to treat an immune-compromised leukemic patient.Blood advances 2018, 2(14): 1691-1695.
. Hombach AA, Heiders J, Foppe M, Chmielewski M, Abken H. OX40 costimulation by a chimeric antigen receptor abrogates CD28 and IL-2 induced IL-10 secretion by redirected CD4(+) T cells. Oncoimmunology 2012, 1(4): 458-466.
. Shen CJ, Yang YX, Han EQ, Cao N, Wang YF, Wang Y, et al. Chimeric antigen receptor comprising ICOS signaling domain mediates specific and efficient antitumor effect of T cells against EGFRvIII expressing glioma. Journal of hematology &oncology 2013, 6: 33.
. Lonez C, Verma B, Hendlisz A, Aftimos P, Awada A, Van Den Neste E, et al. Study protocol for THINK: a multinational open-label phase I study to assess the safety and clinical activity of multiple administrations of NKR-2 in patients with different metastatic tumour types. BMJ open 2017, 7(11): e017075.
. Nair S, Wang JB, Tsao ST, Liu Y, Zhu W, Slayton WB, et al. Functional Improvement of Chimeric Antigen Receptor Through Intrinsic Interleukin-15Ralpha Signaling.Current gene therapy 2018.
. Mata M, Gerken C, Nguyen P, Krenciute G, Spencer DM, Gottschalk S. Inducible Activation of MyD88 and CD40 in CAR T Cells Results in Controllable and Potent Antitumor Activity in Preclinical Solid Tumor Models. Cancer discovery 2017, 7(11):1306-1319.
. Lai Y, Weng J, Wei X, Qin L, Lai P, Zhao R, et al. Toll-like receptor 2 costimulation potentiates the antitumor efficacy of CAR T Cells. Leukemia 2018, 32(3): 801-808.
. Cheng Z, Wei R, Ma Q, Shi L, He F, Shi Z, et al. In Vivo Expansion and Antitumor Activity of Coinfused CD28- and 4-1BB-Engineered CAR-T Cells in Patients with B Cell Leukemia. Molecular therapy: the journal of the American Society of GeneTherapy 2018, 26(4): 976-985.
. Zhang CC, Wang Z, Yang Z, Wang ML, Li SQ, Li YY, et al. Phase I Escalating-DoseTrial of CAR-T Therapy Targeting CEA(+) Metastatic Colorectal Cancers. MolecularTherapy 2017, 25(5): 1248-1258.
. Ahmed N, Brawley VS, Hegde M, Robertson C, Ghazi A, Gerken C, et al. Human Epidermal Growth Factor Receptor 2 (HER2) -Specific Chimeric Antigen Receptor-Modified T Cells for the Immunotherapy of HER2-Positive Sarcoma. Journalof clinical oncology: official journal of the American Society of Clinical Oncology 2015,33(15): 1688-1696.
. Garfall AL, Maus MV, Hwang WT, Lacey SF, Mahnke YD, Melenhorst JJ, et al.Chimeric Antigen Receptor T Cells against CD19 for Multiple Myeloma. The NewEngland journal of medicine 2015,373(11): 1040-1047.
. Wei G, Ding L, Wang J, Hu Y, Huang H. Advances of CD19-directed chimeric antigen receptor-modified T cells in refractory/relapsed acute lymphoblastic leukemia.Experimental hematology & oncology 2017, 6: 10.
. Sadelain M, Riviere I, Riddell S. Therapeutic T cell engineering. Nature 2017,545(7655): 423-431.
. Brown GD, Willment JA, Whitehead L. C-type lectins in immunity and homeostasis.Nature reviews Immunology 2018, 18(6): 374-389.
. Kumaresan PR, Manuri PR, Albert ND, Maiti S, Singh H, MiT, et al. Bioengineering Tcells to target carbohydrate to treat opportunistic fungal infection. Proceedings of theNational Academy of Sciences of the United States of America 2014, 111(29):10660-10665.
. Brown GD, Crocker PR. Lectin Receptors Expressed on Myeloid Cells. Microbiologyspectrum 2016, 4(5).
. Chiba S, Ikushima H, Ueki H, Yanai H, Kimura Y, Hangai S, et al. Recognition of tumor cells byDectin-1 orchestrates innate immune cells for anti-tumor responses. eLife2014, 3: e04177.
. Kaisar MMM, Ritter M, Del Fresno C, Jonasdottir HS, van der Ham AJ, Pelgrom LR, etal.Dectin-1/2-induced autocrine PGE2 signaling licenses dendritic cells to prime Th2responses. PLoS biology 2018, 16(4): e2005504.
. Zhao Y, Chu X, Chen J, Wang Y, Gao S, Jiang Y, et al. Dectin-1-activated dendritic cells trigger potent antitumour immunity through the induction of Th9 cells. Naturecommunications 2016, 7: 12368.
. Rimawi MF, Schiff R, Osborne CK. Targeting HER2 for the treatment of breast cancer.Annual review of medicine 2015, 66: 111-128.
. Mardiana S, John LB, Henderson MA, Slaney CY, von Scheidt B, Giuffrida L, et al. AMultifunctional Role for Adjuvant Anti-4-1BB Therapy in Augmenting Antitumor Response by Chimeric Antigen Receptor T Cells.Cancer research 2017, 77(6):1296-1309.
. Chacon JA, Wu RC, Sukhumalchandra P, Molldrem JJ, Sarnaik A, Pilon-Thomas S, etal. Co-stimulation through 4-1BB/CD137 improves the expansion and function of CD8(+) melanoma tumor-infiltrating lymphocytes for adoptive T-cell therapy. PloS one2013, 8(4): e60031.
. Abken H. Driving CARs on the Highway to Solid Cancer: Some Considerations on theAdoptive Therapy with CAR T Cells. Human gene therapy 2017, 28(11): 1047-1060.
. Bagley SJ, O'Rourke DM. Clinical investigation of CAR T cells for solid tumors: Lessons learned and future directions. Pharmacology & therapeutics 2019: 107419.
. Klebanoff CA, Gattinoni L, Torabi-Parizi P, Kerstann K, Cardones AR, Finkelstein SE,et al. Central memory self/tumor-reactive CD8+ T cells confer superior antitumor immunity compared with effector memory T cells. Proceedings of the National Academy of Sciences of the United States of America 2005, 102(27): 9571-9576.
. Ma Q, Gomes EM, Lo AS, Junghans RP. Advanced generation anti-prostate specific membrane antigen designer T cells for prostate cancer immunotherapy.The Prostate2014, 74(3): 286-296.
. Till BG, Jensen MC, Wang J, Chen EY, Wood BL, Greisman HA, et al. Adoptive immunotherapy for indolent non-Hodgkin lymphoma and mantle cell lymphoma using genetically modified autologous CD20-specific T cells. Blood 2008, 112(6):2261-2271.
. Till BG, Jensen MC, Wang J, Qian X, Gopal AK, Maloney DG, et al. CD20-specificadoptive immunotherapy for lymphoma using a chimeric antigen receptor with bothCD28 and 4-1BB domains: pilot clinical trial results. Blood 2012, 119(17): 3940-3950.
. Muranski P, Borman ZA, Kerkar SP, Klebanoff CA, Ji Y, Sanchez-Perez L, et al. Th17cells are long lived and retain a stem cell-like molecular signature. Immunity 2011,35(6): 972-985.
. Zhang PF, Huang Y, Liang X, Li D, Jiang L, Yang X, et al. Enhancement of the antitumor effect of HER2-directed CAR-T cells through blocking epithelial-mesenchymal transition in tumor cells.FASEB journal: official publication ofthe Federation of American Societies for Experimental Biology 2020, 34(8):11185-11199.
. Gattinoni L, Lugli E, Ji Y, Pos Z, Paulos CM, Quigley MF, et al. A human memory Tcell subset with stem cell-like properties.Nature medicine 2011, 17(10): 1290-1297.

## Claims

1. A chimeric antigen receptor, comprising an extracellular domain, a transmembrane domain and an intracellular domain, wherein the transmembrane domain and the intracellular domain of the chimeric antigen receptor form a costimulatory signaling domain, and the costimulatory signaling domain comprises a full length or a fragment of an amino acid sequence encoding a reverse dectin-1.

2. The chimeric antigen receptor according to claim 1, wherein the extracellular domain comprises a single-chain antibody targeting to CD19 or HER2.

3. The chimeric antigen receptor according to claim 2, wherein the chimeric antigen receptor further comprises a CD8α hinge region, a reverse dectin-1 transmembrane domain, a reverse dectin-1 intracellular signaling domain and a CD3ζ intracellular signaling domain which are connected in sequence.

4. The chimeric antigen receptor according to claim 1, wherein the amino acid sequence encoding the reverse dectin-1 is shown in SEQ ID NO:3.

5. The chimeric antigen receptor according to claim 3, wherein an amino acid sequence encoding the reverse dectin-1 transmembrane domain is shown in SEQ ID NO:2.

6. The chimeric antigen receptor according to claim 3, wherein an amino acid sequence encoding the reverse dectin-1 intracellular signaling domain is shown in SEQ ID NO:1.

7. A lentiviral vector comprising the chimeric antigen receptor according to any one of claims 1-6.

8. The lentiviral vector according to claim 7, wherein the lentiviral vector comprises pCLK, psPAX2 or pMD2.0G.

9. A CAR-T cell, wherein expressing the chimeric antigen receptor according to any one of claims 1-6.

10. An antitumor drug, comprising the CAR-T cell according to claim 9 and a pharmaceutically acceptable excipient and/or adjuvant.

11. The antitumor drug according to claim 10, wherein the tumor comprises hematological tumors and solid tumors.

12. The antitumor drug according to claim 10, wherein the tumor comprises large B-cell lymphoma, B-cell lymphoma, non-Hodgkin lymphoma, myelogenous leukemia, lymphoblastic leukemia, breast cancer, gastric cancer, esophageal cancer or ovarian cancer.

13. Use of the CAR-T cell according to claim 9 in preparation of an antitumor drug.

14. The use according to claim 13, wherein the CAR-T cell can stimulate secretion of effector cytokine.

15. The use according to claim 14, wherein the effector cytokine comprises IFN-γ, TNF-α and IL-6.

16. The use according to claim 13, wherein the CAR-T cell, upon being stimulated, display phenotype of a central memory T cell.

17. The use according to claim 13, wherein the tumor comprises hematological tumors and solid tumors.

18. The use according to claim 13, wherein the tumor comprises large B-cell lymphoma, B-cell lymphoma, non-Hodgkin lymphoma, myelogenous leukemia, lymphoblastic leukemia, breast cancer, gastric cancer, esophageal cancer or ovarian cancer.

19. A synthesis method for the chimeric antigen receptor according to any one of claims 1-6, comprising the following steps: (1) synthesizing a gene sequence of the reverse dectin-1 transmembrane domain - reverse dectin-1 intracellular domain or a gene sequence of the reverse dectin-1 transmembrane domain - CD3ζ intracellular signaling domain; (2) synthesizing primers according to a target, and synthesizing a gene sequence of the chimeric antigen receptor by an overlapping PCR method.

20. The synthesis method according to claim 19, wherein the step (2) specifically comprises: first using primers F1 and R1 to expand a bound gene sequence of the extracellular domain and the CD8α hinge region, then using primers F2 and R2 to expand the gene sequence of the reverse dectin-1 transmembrane domain - CD3ζ intracellular signaling domain, and finally using the bound gene sequence of the extracellular domain and the CD8α hinge region as well as the gene sequence of the reverse dectin-1 transmembrane domain - CD3ζ intracellular signaling domain as templates and using F1 and R2 as primers to synthesize the gene sequence of the chimeric antigen receptor.

21. The synthesis method according to claim 20, wherein the primer F1 is shown in SEQ ID NO:7, R1 is shown in SEQ ID NO:8, F2 is shown in SEQ ID NO:9, and R2 is shown in SEQ ID NO:10.
